(19) Europäisches Patentamt
European Patent Office
Office européen des brevets

(11) Veröffentlichungsnummer: **0 320 769 A2**

(12) # EUROPÄISCHE PATENTANMELDUNG

(21) Anmeldenummer: **88120433.3**

(22) Anmeldetag: **07.12.88**

(51) Int. Cl.[4]: **C07C 93/16 , C08G 65/32 , C23F 11/14**

(30) Priorität: **18.12.87 DE 3742935**

(43) Veröffentlichungstag der Anmeldung: **21.06.89 Patentblatt 89/25**

(84) Benannte Vertragsstaaten: **DE GB NL**

(71) Anmelder: **HOECHST AKTIENGESELLSCHAFT**
**Postfach 80 03 20**
**D-6230 Frankfurt am Main 80(DE)**

(72) Erfinder: **Wirtz, Herbert, Dr.**
**Rossertstrasse 31**
**D-6239 Eppstein/Taunus(DE)**
Erfinder: **Hoffmann, Hermann, Dr.**
**In den Padenwiesen 13**
**D-6233 Kelkheim (Taunus)(DE)**
Erfinder: **Ritschel, Werner, Dr.**
**Kohlweg 11**
**D-6240 Königsteim (Taunus)(DE)**
Erfinder: **Hofinger, Manfred, Dr.**
**Rossfeldstrasse 7a**
**D-8269 Burgkirchen(DE)**
Erfinder: **Mitzlaff, Michael, Dr.**
**Brüningstrasse 33a**
**D-6380 Bad Homburg(DE)**
Erfinder: **Wolter, Dietrich**
**Höhenweg 8**
**D-6237 Liederbach(DE)**

(54) **Gegebenenfalls quaternierte Fettsäureester von oxyalkylierten Alkyl-alkylendiaminen.**

(57) Gegebenenfalls quaternierte Ester von oxalkylierten Alkyl-alkylendiaminen der Formel

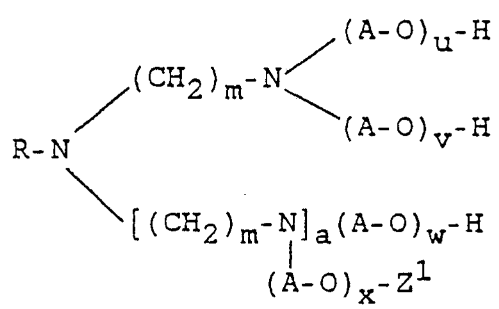

wobei die einzelnen Symbole in der Beschreibung aufgeführte Bedeutung haben, Verfahren zu deren Herstellung und deren Verwendung als Korrosionsschutzmittel, insbesondere in Erdölgewinnungs- und Verarbeitungsanlagen.

EP 0 320 769 A2

## Gegebenenfalls quaternierte Fettsäureester von oxalkylierten Alkyl-alkylendiaminen

In technischen Prozessen, bei denen Metalle mit Wasser oder auch mit Öl-Wasser-Zweiphasensystemen in Kontakt kommen, besteht die Gefahr der Korrosion. Besonders in Salzwassersystemen, wie sie in Erdölgewinnungs- und -verarbeitungsprozessen vorkommen, ist diese Gefahr sehr hoch. Ohne spezielle Mittel zum Schutze der eingesetzten Ausrüstungen ist die Ausbeute einer Lagerstätte und die Verarbeitung des Erdöles praktisch unmöglich.

Solche Korrosionsschutzmittel sind z.B. die in US 2 940 927 aufgeführten Aminoxethylate, die aber in manchen Fällen noch nicht genügend wirksam sind. Weiterhin sind in US 4 010 111 Fettsäureamide als Korrosionsinhibitoren für sauerstoffhaltige Systeme und in US 3 997 469 Amide auf Basis von Naphthensäure beschrieben. Diese Mittel sind aber überhaupt nicht oder nur schwer löslich, was ihre Wirksamkeit erheblich einschränkt, weil diese Stoffe sich dann in der Ölphase befinden und das Salzwasser demzufolge das Eisen ungehindert angreifen kann. Aus JP-B 68/03 001 (Chem. Abstr. 69 (1968), 20023n) sind oxalkylierte Alkylalkylendiamine und deren Wirkung als Korrosionsschutzmittel bekannt. Die Wirkung dieser Verbindungen ist jedoch weniger gut als bei den im folgenden beschriebenen Veresterungsprodukten

Das Ziel der Erfindung bestand deshalb darin, solche Verbindungen herzustellen, die als Korrosionsinhibitoren in solchen Wasser-in-Öl-Emulsionen wirksam sind, wie sie bei der Erdölförderung oder -verarbeitung vorkommen.

Ein weiteres Ziel bestand darin, solche Korrosionsinhibitoren aufzufinden, die auch in hochkonzentrierter Salzlösung zumindestens kolloiddispers verteilt sind.

Gegenstand der Erfindung sind gegebenenfalls quaternierte Ester von oxalkylierten Alkyl-alkylendiaminen, erhalten durch Veresterung von oxalkylierten Alkyl-alkylendiaminen der Formel

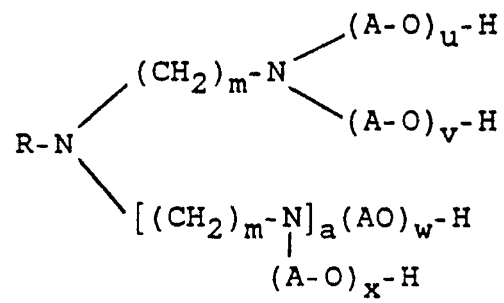

wobei R $C_8$-$C_{24}$-Alkyl oder $C_8$-$C_{24}$-Alkenyl, A eine Gruppe der Formel $-C_2H_4-$ oder $-C_3H_6-$ bedeuten, a 0 oder 1 ist, m 2 oder 3 ist, vorzugsweise 3, u, v, w und x Zahlen sind, deren Summe für den Fall a = 0 3 bis 30 und für den Fall a = 1 4 bis 40 betragen, mit Säuren der Formel

$HOOC-R^1-(COOH)_n$

wobei n = 0 oder 1 ist und $R^1$ für den Fall n = 0 Alkyl, Alkenyl, Cycloalkyl oder Cycloalkenyl mit jeweils 5 bis 35 C-Atomen oder $R^1$ für den Fall n = 1 den Rest einer dimeren Fettsäure bedeutet, und gegebenenfalls anschließende Quaternierung unter Bildung von Gruppierungen der Formel $\overset{\oplus}{N}-R^2\ M^{\ominus}$, wobei $R^2$ $C_1$-$C_4$-Alkyl oder Benzyl und $M^-$ ein Anion bedeuten, ausgenommen solche Verbindungen die durch Veresterung von oxalkylierten Alkyl-alkylendiaminen der Formel

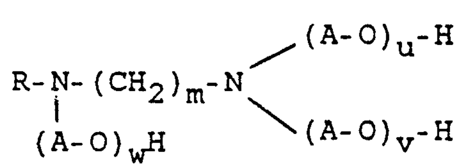

erhalten werden und nicht quaterniert sind.

Bevorzugt sind solche Ester, die durch Veresterung mit $C_{16}$-$C_{22}$-Fettsäuren, insbesondere mit Stearinsäure, oder mit Tallölfettsäure, Naphthensäure oder einer Dimerfettsäure erhalten werden. Der Oxethylierungsgrad liegt bevorzugt bei 10 bis 30 für den Fall a = 0 und bei 20 bis 40 für den Fall a = 1.

Weiterhin ist besonders bevorzugt ein Oxethylierungsgrad von 15 bis 25 im Fall a = 0, im Fall a = 1 ein

Oxethylierungsgrad von 20 bis 30. Als Gruppe -A-O kommen Ethoxy und Propoxy sowie deren Mischungen in Frage, bevorzugt ist $-C_2H_4O-$. Als Anion M ist bevorzugt ein Halogenid-Anion, insbesondere Chlorid, ein Methosulfat- der Methophosphat-Anion.

Die erfindungsgemäßen Ester erhält man durch Umsetzung von oxethylierten Alkyl-alkylendiaminen der Formel

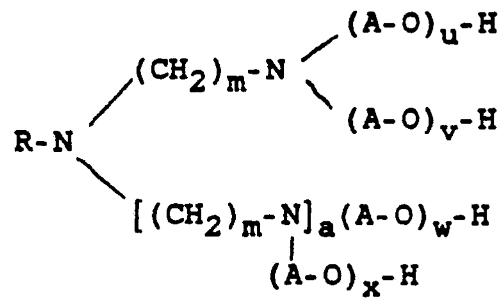

mit einer Säure der angegebenen Formel. Die Umsetzung erfolgt in Substanz oder in hochsiedenden aromatischen Lösungsmitteln mit p-Toluolsulfonsäure als Katalysator bei Temperaturen zwischen 100 °C und 200 °C. Das Molverhältnis Monocarbonsäure : Amin-Oxethylat beträgt dabei 1 : 1 bis 4 : 1. Dementsprechend werden dann eine, mehrere oder sämtliche -(A-O)-H-Gruppen verestert. Bei den Dicarbonsäuren beträgt das Molverhältnis Dicarbonsäure zu Aminoxethylat 0,5 : 1 bis 1,5 : 1.

Die als Ausgangsverbindungen eingesetzten oxalkylierten Alkyl-alkylendiamine erhält man unter den üblichen Bedingungen bei ca. 120 °C bis 1260 °C aus dem Amin und Alkylenoxid unter basischer Katalyse. Die Quaternierung zur Einführung des Restes $R^2$ erfolgt nach bekannten Verfahren, beispielsweise mit Methylchlorid, Benzylchlorid oder Dimethylsulfat in Wasser oder in Isopropanol.

Gegenstand der Erfindung ist weiterhin die Verwendung der oben beschriebenen Ester von oxalkylierten Alkyl-alkylendiaminen als Korrosionsschutzmittel unter Einschluß derjenigen Verbindungen, die durch Veresterung von oxalkylierten Alkyl-alkylendiaminen der Formel

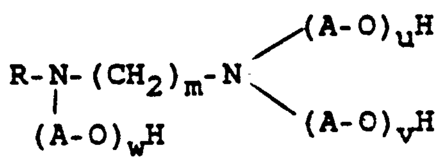

erhalten werden und nicht quaterniert sind.

Insbesondere eignen sich diese Verbindungen als Korrosionsinhibitoren in Erdölgewinnungs- und Verarbeitungsanlagen, die mit Salzwasser in Berührung kommen. Die Einsatzmengen dieser Verbindungen als Korrosionsinhibitoren betragen 5 bis 200, vorzugsweise 5 bis 50 ppm in dem flüssigen Medium.

Die folgenden Beispiele sollen die Erfindung näher erläutern. Die Bestimmung des Oxethylierungsgrades erfolgt durch Titration des basischen Stickstoffs und durch bestimmung der OH-Zahl. Der Fortschritt der Veresterung kann über die abgeschiedene Wassermenge wie auch über die Säurezahl verfolgt werden.

**Beispiel 1**

In einem 1 Liter-4-Halskolben mit Rührer, Innenthermometer und Destillationsbrücke werden 156.8 g (0, 2 mol) des Additionsproduktes aus 1 mol Talgfettpropylendiamin und 10 mol Ethylenoxid mit dem Molekulargewicht 784 vorgelegt. Unter Rühren werden über einen Zeitraum von 10 min 171 g (0, 6 mol) Tallölfettsäure vom Molekulargewicht 285 zugegeben. Man wartet die exotherme Reaktion ab und heizt auf 155 bis 180 °C. Im Verlauf von 6 h destillieren etwa 6 bis 10 ml Wasser ab. Die Säurezahl fällt dabei auf <10 ab. Man erhält eine braune viskose Flüssigkeit.

**Beispiel 2**

In einem 1 Liter-4-Halskolben mit Rührer, Innenthermometer und Wasserabscheider werden 153,8 g (0,1 mol) des Reaktionsproduktes aus Talgfettpropylendiamin und 30 mol Ethylenoxid mit dem Molekulargewicht 1538 in 240 ml eines aromatischen Kohlenwasserstoffs vorgelegt. Dazu gibt man über eine Zeitraum von 10 min 85,5 g (0,15 mol) Dimerfettsäure vom Molekulargewicht 570 zu, wartet die exotherme Reaktion ab und erhitzt anschließend auf 155° C bis 180° C. Im Verlaufe von 6 bis 8 h destilliert man 5 ml Wasser an und erhält ein braunes viskoses Produkt.

**Beispiel 3**

Nach dem in Beispiel 1 angeführten Verfahren werden 123 g (0,1 mol) des Additionsproduktes aus Cocos-bis-(3-aminopropyl)-amin und 20 mol Ethylenoxid vorgelegt. Man erwärmt auf 50 bis 60° C, gibt portionsweise 108 g (0,4 mol) geschuppte Stearinsäure vom Molekulargewicht 270 zu. wartet die exotherme Reakion ab und erhitzt anschließend auf 155 bis 180° C und destilliert über 6 bis 8 h 5 ml Wasser ab. Man erhält ein braunes viskoses Produkt.

**Beispiel 4**

Nach dem in Beispiel 1 angeführten Verfahren werden 220 g (0,1 mol) des Additionsproduktes von Talg-bis-(3-aminopropyl)-amin und 40 mol Ethylenoxid vorgelegt und mit 29,6 g (0,1 mol) Naphthensäure versetzt. Nach Abklingen der exothermen Reaktion wird 6 bis 8 h bei 155 bis 180° C gehalten, wobei ca. 2 ml Wasser übergehen. Die Säurezahl fällt dabei unter 3. Man erhält eine braune, viskose Flüssigkeit.

**Beispiel 5**

320 g des nach Beispiel 1 hergestellten Aminoxethylatesters werden unter Rühren mit 345 g Wasser versetzt und auf 80° C erwärmt. Zu dieser Lösung tropft man während 30 min, 50,6 g (0,4 mol) Benzylchlorid. Nach beendeter Zugabe wird noch 6 h bei Rückflußtemperatur gehalten.

**Beispiel 6**

226 g des nach Beispiel 3 hergestellten Aminoxethylatesters werden mit 60 g Isopropanol versetzt und im Autoklaven nach den üblichen Bedingungen bei 60° C mit Methylchlorid quaterniert.

Die nach den Beispielen 1 bis 6 erhaltenen Produkte haben folgende Struktur:

**Beispiel 1:**

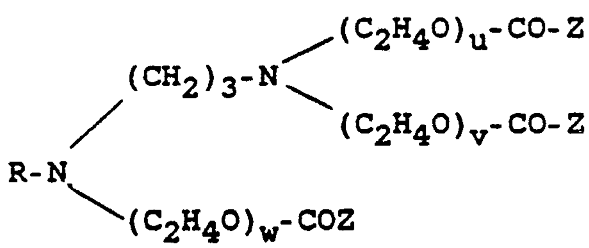

**R= Talgfettalkyl, Z= Tallölfettsäurealkyl, Summe u, v und w= 10.**

**Beispiel 2:**

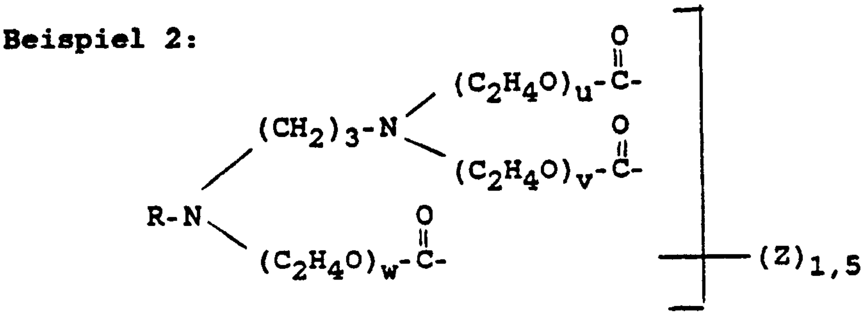

R= Talgfettalkyl, Z= Dimerfettsäurealkyl, Summe u, v und w= 30.

**Beispiel 3:**

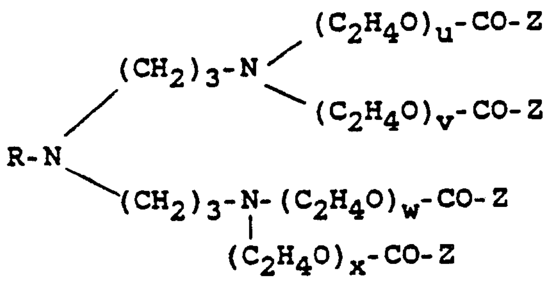

R= Cocosfettalkyl, Z= Stearinsäurealkyl, Summe u, v, w und z= 20.

**Beispiel 4:**

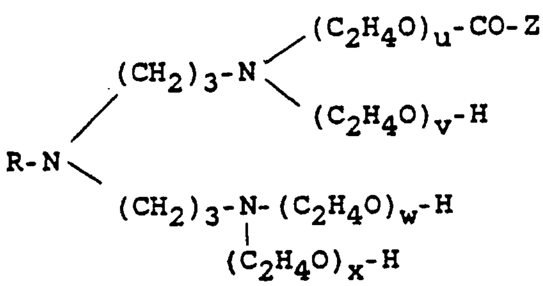

R= Talgfettalkyl, Z= Naphthensäurealkyl, Summe u, v, w und x= 40.

**Beispiel 5:**

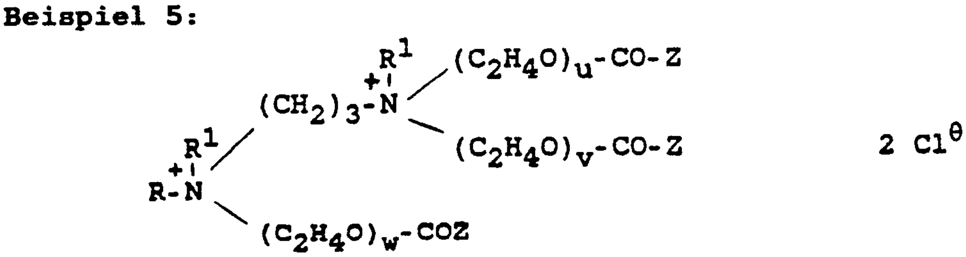

**R = Talgfettalkyl, Z= Tallölfettsäurealkyl, Summe u, v und w= 10, $R^1$= Benzyl.**

**Beispiel 6:**

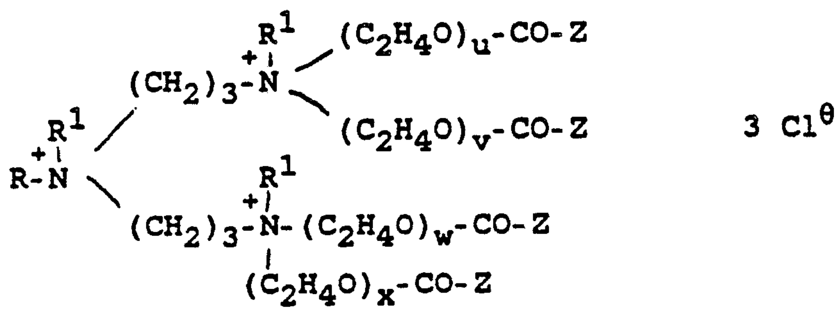

**R = Cocosfettalkyl, Z= Stearinsäurealkyl, Summe u, v, w und z= 20, $R^1$= Methyl.**

Zur Prüfung der Inhibitorkompositionen wurde ein dynamischer Test (sog. "Wheel-Test") herangezogen, eine Methode, mit der die Korrosionsinhibitoren für die Erdöl- und Erdgasförderung getestet werden. Eine Beschreibung der Methode ist in der Zeitschrift Materials Performance 21, Dec. 1982, pp. 45 bis 47 zu finden.

Als Testcoupons wurden Stahlbleche der Abmessungen 75 mm x 10 mm x 1 mm gewählt. Die Blechstreifen wurden geschmirgelt, mit Aceton entfettet und gewogen. Als Testmedium diente Salzwasser mit 5 % gelöstem NaCl, dem außerdem 10 Vol-% frisch destilliertes Kerosin zugemischt wurde. Der pH der wäßrigen Lösung war im Falle der $CO_2$-Begasung mit Essigsäure auf pH = 3,5 abgesenkt.

Beide Phasen wurden vor beginn eines Korrosionstests mit Stickstoff eine halbe Stunde durchgeperlt, um gelösten Sauerstoff zu vertreiben. Danach wurden beide Phasen separat entweder mit gasförmigem $CO_2$ bzw. $H_2O$ bis zur Sättigung, aber mindestens 30 min durchströmt.

Dann wurden 10,20 und 50 ppm - bezogen auf das Volumen des Testmediums - an Inhibitor zugesetzt.

Die entfetteten und gewogenen Bleche wurden anschließend in die Emulsionen eingebracht und bei 70° C 24 Stunden einer mechanischen Bewegung (40 Upm mittels einer die Testgefäße drehenden Welle) unterzogen.

Die Testblechstreifen wurden anschließend mechanisch gereinigt und nach Trocknung zur Bestimmung des Gewichtsverlustes gewogen. Die Korrosionsraten sind im mpy (mils per year) angegeben (39,4 mpy = 1 mm Jahr). Zum Vergleich wurde der Blindwert (Versuch ohne Inhibitorzusatz ermittelt.

Die mit dieser Testmethode erhaltenen Ergebnisse sind in der folgenden Tabelle zusammengefaßt. Die Tabelle enhält die aus Gewichtsabträgen bestimmten Schutzwerte der in Gegenwart der in den Beispielen 1 bis 6 beschriebenen Verbindungen. Zum Vergleich sind Daten für ein marktgängiges Produkt der Fa. Servo aufgeführt. Ein Vergleich der Schutzwerte belegt die Überlegenheit der erfindungsgemäß beschriebenen Produkte. Die in der folgenden Tabelle aufgeführten Schutzwerte beziehen sich auf die Blindwerte der Korrosion von 0,85 mm/a für den Fall der $CO_2$-Korrosion bzw. 0,71 mm/a für dem Fall der $H_2S$-Korrosion.

| | $CO_2$ | | | $H_2S$ | | |
|---|---|---|---|---|---|---|
| | 5 | 10 | 50 ppm | 5 | 10 | 50 ppm |
| Beispiel 1 | 61 | 78 | 82 | 77 | 80 | 93 |
| 2 | 71 | 79 | 80 | 68 | 77 | 86 |
| 3 | 68 | 82 | 83 | 73 | 82 | 95 |
| 4 | 58 | 64 | 78 | 83 | 86 | 94 |
| 5 | 64 | 73 | 76 | 64 | 77 | 91 |
| 6 | 75 | 78 | 84 | 68 | 74 | 96 |
| Servo CK 378 | 48 | 59 | 71 | 57 | 65 | 70 |

**Ansprüche**

1. Gegebenenfalls quaternierte Ester von oxalkylierten Alkyl-alkylendiaminen, erhalten durch Veresterung von oxalkylierten Alkyl-alkylendiaminen der Formel

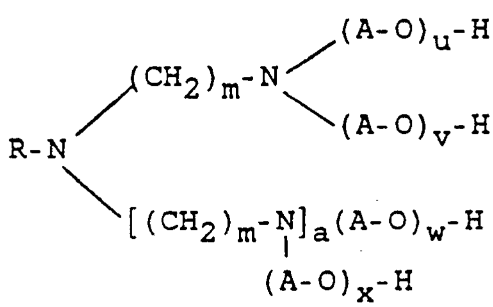

wobei R $C_8$-$C_{24}$-Alkyl oder $C_8$-$C_{24}$-Alkenyl, A eine Gruppe der Formel $-C_2H_4-$ oder $-C_3H_6-$ bedeuten, a 0 oder 1 ist, m 2 oder 3 ist, u, v, w und x Zahlen sind, deren Summe für den Fall a=0 3 bis 30 und für den Fall a=1 4 bis 40 betragen, mit Säuren der Formel
$HOOC-R'-(COOH)_n$
wobei n 0 oder 1 ist und R' für den Fall n= 0 Alkyl, Alkenyl, Cycloalkyl oder Cycloalkenyl mit jeweils 5 bis 35 C-Atomen oder R' für den Fall n= 1 den Rest einer dimeren Fettsäure bedeutet, und gegebenenfalls anschließende Quaternierung unter Bildung von Gruppierungen der Formel $\overset{\oplus}{N}-R^2 M^{\theta}$, wobei $R^2$ $C_1$-$C_4$-Alkyl oder Benzyl und $M^-$ ein Anion bedeuten, ausgenommen solche Verbindungen die durch Veresterung von oxalkylierten Alkyl-alkylendiaminen der Formel

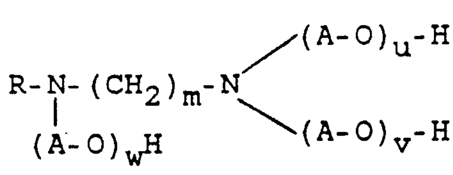

erhalten werden und nicht quaterniert sind.

2. Gegebenenfalls quaternierte Ester von oxalkylierten Alkyl-alkylendiaminen nach Anspruch 1, dadurch gekennzeichnet, daß sie durch Umsetzung mit $C_{16}$-$C_{22}$-Fettsäuren, Tallölfettsäure oder Naphthensäure oder Dimerfettsäure erhalten werden.

3. Gegebenenfalls quaternierte Ester von oxalkylierten Alkyl-alkylendiaminen nach Anspruch 1, dadurch gekennzeichnet, daß M ein Halogenid, Methosulfatoder Methophosphat-Ion bedeutet.

4. Gegebenenfalls quaternierte Ester von oxalkylierten Alkyl-alkylendiaminen nach Anspruch 1, dadurch gekennzeichnet, daß für den Fall a = 0 die Summe der Zahlen u, v, w 10 bis 30 und für den Fall a = 1 die Summe der Zahlen u, v, w und x 20 bis 40 beträgt.

5. Gegebenenfalls quaternierte Ester von oxalkylierten Alkyl-alkylendiaminen nach Anspruch 1, dadurch gekennzeichnet, daß für den Fall a = 0 die Summe der Zahlen u, v, w 15 bis 25 und für den Fall a = 1 die Summe der Zahlen, u, v, w und x 20 bis 30 beträgt.

6. Verwendung von gegebenenfalls quaternierten Estern von oxalkylierten Alkyl-alkylendiaminen, erhalten durch Veresterung von oxalkylierten Alkyl-alkylendiaminen der Formel

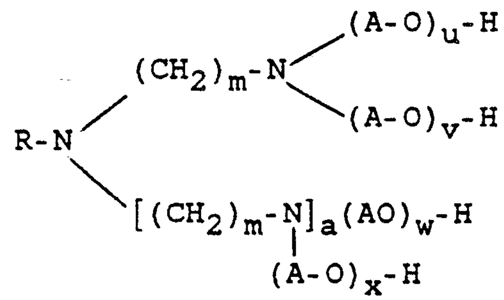

wobei R $C_8$-$C_{24}$-Alkyl oder $C_8$-$C_{24}$-Alkenyl, A eine Gruppe der Formel -$C_2H_4$- oder -$C_3H_6$- bedeuten, a 0 oder 1 ist, m 2 oder 3 ist, u, v, w und x Zahlen sind, deren Summe für den Fall a = 0 3 bis 30 und für den Fall a = 1 4 bis 40 betragen, mit Säuren der Formel

$HOOC-R'-(COOH)^n$

wobei n 0 oder 1 und $R^1$ für den Fall n = 0 Alkyl, Alkenyl, Cycloalkyl oder Cycloalkenyl mit jeweils 5 bis 35 C-Atomen oder R' für den Fall n = 1 den Rest einer dimeren Fettsäure bedeuten, und gegebenenfalls anschließende Quaternierung unter Bildung von Gruppierungen der Formel $>\overset{\oplus}{N}-R^2M^{\theta}$, wobei $R^2$ $C_1$-$C_4$-Alkyl oder Benzyl und $M^{\ominus}$ ein Anion bedeuten, als Korrosionsschutzmittel.